# EUROPEAN PATENT SPECIFICATION

(11) **EP 3 448 836 B1**
(45) Date of publication and mention of the grant of the patent: **13.01.2021**
(21) Application number: 17721254.5
(22) Date of filing: 20.04.2017
(51) Int. Cl.: C07D 209/48, B01D 11/04, C08G 73/10, C08L 79/08

(54) **METHODS AND SYSTEMS FOR THE MANUFACTURE OF AN AROMATIC PHTHALIC BISIMIDE AND A POLYETHERIMIDE**
VERFAHREN UND SYSTEME ZUR HERSTELLUNG EINES AROMATISCHEN PHTHALBISIMIDS UND EINES POLYETHERIMIDS
PROCÉDÉS ET SYSTÈMES POUR LA PRODUCTION D'UN BISIMIDE PHTALIQUE AROMATIQUE ET D'UN POLYÉTHERIMIDE

(30) Priority: 29.04.2016 US 201662329643 P
(43) Date of publication of application: 06.03.2019
(73) Proprietor: SHPP Global Technologies B.V., 4612 PX Bergen op Zoom (NL)
(72) Inventor: CROLL, Joshua McClellan, Mount Vernon, Indiana 47620-9367 (US); ALJARRAH, Mohannad, Mount Vernon, Indiana 47620-9367 (US); GUGGENHEIM, Thomas Link, Mount Vernon, Indiana 47620-9367 (US)
(74) Representative: Office Freylinger
(86) International application number: PCT/US2017/028541
(87) International publication number: WO 2017/189314

(56) References cited:
- WO-A1-00/14125
- WO-A1-2009/143440
- US-A- 5 132 423
- Various authors: "Ullmann's Encyclopedia of Industrail Chemistry 6th Edition", 2003, XP002770559, ISBN: 978-3-527-30673-2 vol. 19, pages 673-726, page 675, paragraph Applications of Liquid-Liquid Extraction page 687, paragraph Apparatus - page 693 Change in temperature; page 713, paragraph

## Description

### BACKGROUND

Polyetherimides are a class of high performance polymers that can be processed to make molded articles, fibers, films, foams, and the like. Polyetherimides further have high strength, toughness, heat resistance, modulus, and broad chemical resistance, and so are widely used in industries as diverse as automotive, telecommunication, aerospace, electrical/electronics, transportation, and healthcare. Polyetherimides have shown versatility in various manufacturing processes, proving amenable to techniques including injection molding, extrusion, and thermoforming, to prepare various articles.

However, some polyetherimides do not meet stringent purity requirements necessary for some applications, for example, the polyetherimides can be required to have very low contaminant levels, or the processability and product performance is adversely affected. Common resin contaminants could be organic or inorganic in nature. The organic contaminants are mostly lower molecular weight species, including residual phenolic monomers or derivatives thereof. Besides affecting polymer properties, residual monomers can also be of concern in view of emerging regulatory considerations.

WO 2009/143440 A1 describes that the production of low color polyetherimides, including their intermediates, such as bisimides and diaryl diether dianhydrides, may be affected by producing an improved purity intermediate of 4-nitro-N-alkylphthalimide. A salt, such as alkali metal carbonate or alkali metal hydrogen carbonate, is added to an aqueous mixture of 4-nitro-N-alkylphthalimide and 3-nitro-Nalkylphthalimide to selectively hydrolyze the imide linkage of 3-nitro-Nalkylphthalimide forming a water-soluble acid-amide salt. An organic solvent is added to this salt mixture to phase separate 4-nitro-N-alkylphthalimide having dissolved in the organic solvent from acid-amide salt of 3-nitro-N-alkylphthalimide having dissolved in water.

US5,132,423 discloses reactions between a solid polar and a non-polar compound, especially nucleophilic aromatic substitution reactions between an alkali metal salt of a hydroxyaromatic compound or thio analog thereofand an activated halo- or nitro-substituted aromatic compound, are conducted in a non-polar organic solvent such as toluene or xylene, in the presence of a hexaalkylguanidinium or α,ω)-bis(pentaalkylguanidinium)alkane salt, or a corresponding heterocyclic salt, as a phase transfer catalyst. This method is particularly is said to be useful for the preparation of bisimides from bisphenol A or 4,4'-biphenol salts and 4-nitro- or 4-halophthalimides.

Accordingly, there remains a continuing need for an improved process for producing high quality polyetherimides, specifically polyetherimides having a non-detectable level of residual phenolic monomers or derivatives thereof.

### BRIEF DESCRIPTION

A method for producing an aromatic bisimide comprises reacting a dialkali metal salt of a dihydroxy aromatic compound of the formula

M⁺⁻O-Z-O⁻⁺M

with a reactive substituted phthalimide of the formula 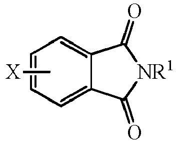 under conditions effective to form a product mixture comprising at least one of the dialkali metal salt of the dihydroxy aromatic compound, a dihydroxy aromatic compound corresponding to the dialkali metal salt of the dihydroxy aromatic compound, a mono-substituted salt of the dihydroxy aromatic compound of the formula 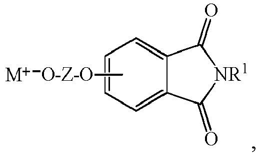 or a combination comprising at least one of the foregoing, and the aromatic bisimide, wherein the aromatic bisimide is of the formula 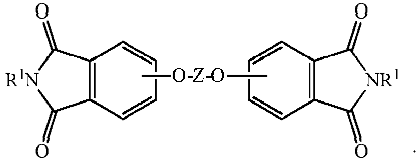
introducing the product mixture to an agitated liquid-liquid extraction column with a 0.01 to 5 weight percent aqueous alkali metal hydroxide solution to extract residual dialkali metal salt, the corresponding dihydroxy aromatic compound, and the corresponding mono-substituted salt of the dihydroxy aromatic compound from the product mixture; and
recovering from the liquid-liquid extraction column a purified aromatic bisimide having less than 500 ppm, or less than 100 ppm, or less than 1 ppm, or less than 10 parts per billion of residual dialkali metal salt, the corresponding dihydroxy aromatic compound, the corresponding mono-substituted salt of the dihydroxy aromatic compound or a combination comprising at least one of the foregoing;
wherein in the foregoing formulas,
M is lithium, sodium, or potassium;
Z is a divalent group of the formula 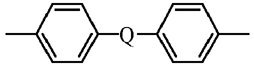
   wherein Q is -CyH2y- wherein y is an integer from 1 to 5 or a halogenated derivative thereof, X is nitro, and
   R¹ is a monovalent C₁₋₄ alkyl group.

An aromatic bisimide comprises less than 500 ppm, or less than 100 ppm, or less than 1 ppm, or less than 10 parts per billion of residual dihydroxy aromatic compound, preferably bisphenol A.

A method for the manufacture of a polyetherimide comprises contacting an aromatic bisimide with a phthalic anhydride in the presence of a catalyst and under conditions effective to provide an aromatic bis(ether phthalic anhydride) of the formula 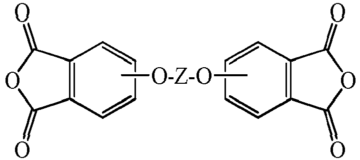 wherein Z is an aromatic C₆₋₂₄ monocyclic or polycyclic moiety optionally substituted with 1 to 6 C₁₋₈ alkyl groups, 1 to 8 halogen atoms, or a combination comprising at least one of the foregoing; and contacting the aromatic bis(ether phthalic anhydride) with an organic diamine of the formula

H₂N-R²-NH₂

wherein R² is an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 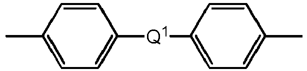 wherein Q¹ is -C_{y}H_{2y}- wherein y is an integer from 1 to 5, and wherein the polyetherimide has less than 10 parts per billion, preferably less than 9 parts per billion of residual dialkali metal salt of a dihydroxy aromatic compound, the corresponding dihydroxy aromatic compound, the corresponding mono-substituted salt of the dihydroxy aromatic compound or a combination comprising at least one of the foregoing.

The above described and other features are exemplified by the following Figures and Detailed Description.

### BRIEF DESCRIPTION OF THE DRAWINGS

The following Figure represents an exemplary embodiment.

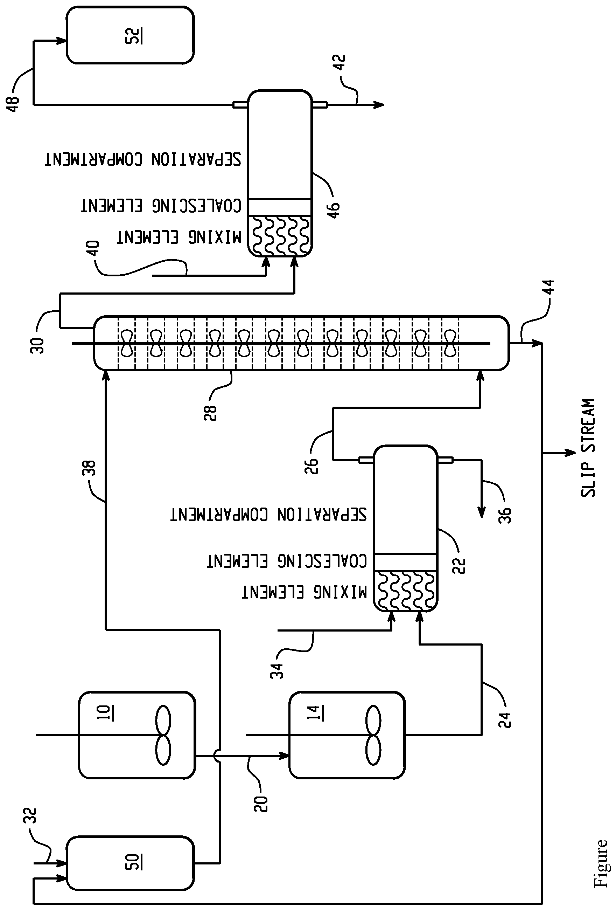

The Figure is a schematic illustration of a system for the manufacture of an aromatic bisimide including a continuous liquid-liquid extraction column.

### DETAILED DESCRIPTION

The scope of the invention is defined by the claims, all other subject matter is seen as forming part only of the disclosure or for reference matters.

Described herein are methods and systems for the manufacture of aromatic bisimides and polyetherimides. The inventors hereof have advantageously discovered that aromatic bisimides can be purified using liquid-liquid extraction techniques that can provide aromatic bisimides having non-detectable levels of residual dihydroxy aromatic compounds (e.g., bisphenol A), specifically less than 500 parts per million (ppm), or less than 100 ppm, or less than 1 ppm, or less than 10 parts per billion (ppb) of residual dihydroxy aromatic compounds. Aromatic bisimides produced according to the methods described herein can be used to produce the corresponding polyetherimides. The polyetherimides having non-detectable levels of residual dihydroxy aromatic compounds can advantageously be used to prepare articles for various applications, particularly in applications where the presence of residual dihydroxy aromatic compounds is of concern in view of customer requirements or regulatory considerations.

One aspect of the present disclosure is a method for producing an aromatic bisimide. The method comprises reacting a dialkali metal salt of a dihydroxy aromatic compound with a reactive substituted phthalimide under conditions effective to form a product mixture comprising at least one of the dialkali metal salt, the corresponding dihydroxy aromatic compound, the corresponding mono-substituted salt of the dihydroxy aromatic compound, or a combination comprising at least one of the foregoing, and the aromatic bisimide. In some embodiments, the product mixture comprises the dialkali metal salt, the corresponding dihydroxy aromatic compound, the corresponding mono-substituted salt of the dihydroxy aromatic compound, and the aromatic bisimide. In some embodiments, the product mixture comprises residual reactive substituted phthalimide, hydrolyzed derivatives thereof, or both.

The dialkali metal salt of the dihydroxy aromatic compound is of formula (1)

M⁺⁻O-Z-O⁻⁺M (1)

wherein M is an alkali metal ion, for example, lithium, sodium, potassium, or a combination comprising at least one of the foregoing. In some embodiments, M is sodium. Z is an aromatic C₆₋₂₄ monocyclic or polycyclic moiety optionally substituted with 1 to 6 C₁₋₈ alkyl groups, 1 to 8 halogen atoms, or a combination comprising at least one of the foregoing. Exemplary Z groups include groups of formula (2) 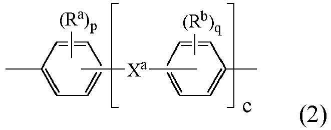 wherein R^{a} and R^{b} are each independently the same or different, and are a halogen atom or a monovalent C₁₋₆ alkyl group, for example; p and q are each independently integers of 0 to 4; c is 0 to 4; and X^{a} is a bridging group connecting the hydroxy-substituted aromatic groups, where the bridging group and the hydroxy substituent of each C₆ arylene group are disposed ortho, meta, or para (specifically para) to each other on the C₆ arylene group. The bridging group X^{a} can be a single bond, -O-, -S-, -S(O)-, -S(O)₂-, -C(O)-, or a C₁₋₁₈ organic bridging group. The C₁₋₁₈ organic bridging group can be cyclic or acyclic, aromatic or non-aromatic, and can further comprise heteroatoms such as halogens, oxygen, nitrogen, sulfur, silicon, or phosphorous. The C₁₋₁₈ organic group can be disposed such that the C₆ arylene groups connected thereto are each connected to a common alkylidene carbon or to different carbons of the C₁₋₁₈ organic bridging group. A specific group Z is a divalent group of formula (2a) 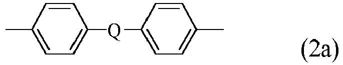 wherein Q is -O-, -S-, -C(O)-, -SO₂-, -SO-, -P(R^{a})(=O)- wherein R^{a} is a C₁₋₈ alkyl or C₆₋₁₂ aryl, or -C_{y}H_{2y}- wherein y is an integer from 1 to 5 or a halogenated derivative thereof. Exemplary dihydroxy aromatic compounds from which Z can be derived include but are not limited to 2,2-bis(2-hydroxyphenyl)propane, 2,4'-dihydroxydiphenylmethane, bis(2-hydroxyphenyl)methane, 2,2-bis-(4-hydroxyphenyl)propane ("bisphenol A" or "BPA"), 1,1-bis-(4-hydroxyphenyl)ethane, 1,1-bis-(4-hydroxyphenyl)propane,2,2-bis-(4-hydroxyphenyl)pentane, 3,3-bis-(4-hydroxyphenyl)pentane, 4,4'-dihydroxybiphenyl, 4,4'-dihydroxy-3,3,5,5 '-tetramethylbiphenyl, 2,4'-dihydroxybenzophenone, 4,4'-dihydroxydiphenylsulfone, 2,4'-dihydroxydiphenylsulfone, 4,4'-dihydroxydiphenylsulfoxide, 4,4'-dihydroxydiphenylsulfide, hydroquinone, resorcinol, 3,4-dihydroxydiphenylmethane, 4,4'-dihydroxybenzophenone, 4,4'-dihydroxydiphenylether, and the like, or a combination comprising at least one of the foregoing. In some embodiments, Z is preferably 2,2-(4-phenylene)isopropylidene (i.e., the dihydroxy aromatic compound from which the dialkali metal salt is derived is 2,2-bis-(4-hydroxyphenyl)propane (bisphenol A).

The reactive substituted phthalimide is of formula (3) 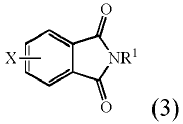 wherein X is fluoro, chloro, bromo, iodo, nitro, or a combination comprising at least one of the foregoing, and R¹ is a monovalent C₁₋₁₃ organic group. In some embodiments, X is nitro. In some embodiments, R¹ is a monovalent C₁₋₁₃ alkyl group, preferably a C₁₋₄ alkyl group, for example a methyl group. In some embodiments, X is nitro and R¹ is a methyl group. In some embodiments, the reactive substituted phthalimide comprises 4-nitro-N-methylphthalimide, 3-nitro-N-methylphthalimide, or a combination comprising at least one of the foregoing.

The product mixture can also include the corresponding mono-substituted salt of the dihydroxy aromatic compound. The mono-substituted salt of the dihydroxy aromatic compound is of formula (4) 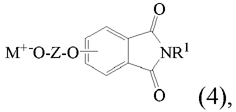 wherein M, Z, and R¹ are as defined above. In some embodiments, M is sodium, Z is 2,2-(4-phenylene)isopropylidene, and R¹ is a methyl group.

In some embodiments, reacting the dialkali metal salt of a dihydroxy aromatic compound with the reactive substituted phthalimide is in the presence of one or both of a solvent and a catalyst. Thus, in addition to comprising at least one of the dialkali metal salts, the corresponding dihydroxy aromatic compound, the corresponding mono-substituted salt of the dihydroxy aromatic compound, or a combination comprising at least one of the foregoing, the product mixture can further include a solvent. Any nonpolar organic solvent which does not react with the reactants during the formation of the aromatic bisimide can be used in the reaction. Preferably, the solvent is not miscible with water. Suitable nonpolar organic solvents include, but are not limited to, toluene, benzene, chlorobenzene, o-dichlorobenzene, 1,2,4-trichlorobenzene, xylene, and the like, or a combination comprising at least one of the foregoing. In some embodiments, the solvent preferably comprises toluene.

In some embodiments, the product mixture further includes a phase transfer catalyst. In some embodiments, the phase transfer catalyst is a hexaalkylguanidinium salt or a tetraalkylammonium salt. For example, the phase transfer catalyst can be hexaethylguanidinium chloride, tetraethylammonium bromide, tetraethylammonium acetate, tetrabutylammonium bromide, and the like. Mixtures of catalysts can also be used. In some embodiments, the phase transfer catalyst is preferably a hexaalkylguanidinium salt, for example hexaethylguanidinium chloride.

The dialkali metal salt of the dihydroxy aromatic compound and the reactive substituted phthalimide can be reacted under any suitable reaction conditions that are generally known in the art. For example, the reacting can be at a temperature of 25 to 180°C, preferably 85 to 125°C, more preferably 100 to 125°C. The reacting is preferably in the presence of a solvent, and in some embodiments, the product mixture can have a solids content of 20 to 50 weight percent, where the term "solids content" is defined as the weight of the dialkali metal salt of the dihydroxy aromatic compound and the reactive substituted phthalimide relative to the total weight of the product mixture. The reacting can also be in the presence of a phase transfer catalyst. The phase transfer catalyst can be present in an amount of 0.3 to 10 mole percent, based on moles of the dialkali metal salt of the dihydroxy aromatic compound. In some embodiments, 0.7 to 1.2 mole percent of the phase transfer catalyst can be used, preferably where the phase transfer catalyst is a hexaalkylguanidinium salt, more preferably wherein the phase transfer catalyst is hexaethylguanidinium chloride. In some embodiments, 1.8 to 2.2 mole percent, preferably 2 mole percent, of the phase transfer catalyst can be used, for example when the phase transfer catalyst is a tetraalkylammonium salt, preferably tetrabutylammonium bromide. The mole ratio of dialkali metal salt of the dihydroxy aromatic compound to the substituted phthalimide can be 1:1.7 to 1:2.3. In some embodiments, however, two moles of the substituted phthalimide per mole of dialkali metal salt is preferred.

The product mixture comprises the aromatic bisimide of formula (5) 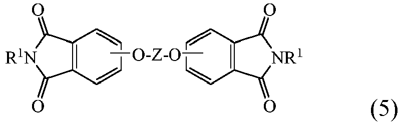 wherein Z and R¹ are as defined above. The divalent bonds of the -O-Z-O- group are in the 3,3', 3,4', 4,3', or the 4,4' positions of the phthalimide phenyl rings. In some embodiments, Z is 2,2-(4-phenylene)isopropylidene, and R¹ is a methyl group. In some embodiments, the aromatic bisimide comprises 4,4'-bisphenol-A-bis-N-methylphthalimide, 3,4'-bisphenol-A-bis-N-methylphthalimide, 3,3'-bisphenol-A-bis-N-methylphthalimide, or a combination comprising at least one of the foregoing.

In some embodiments, the product mixture can further comprise an inorganic alkali metal salt. In some embodiments, the inorganic alkali metal salt can be derived from the reactive substituted phthalimide. For example, when the reactive substituted phthalimide comprises a nitro-substituted N-(C₁₋₁₃ alkyl)phthalimide (e.g., 4-nitro-N-methylphthalimide, 3-nitro-N-methylphthalimide, or a combination comprising at least one of the foregoing), the product mixture can further comprise an alkali metal nitrite (e.g., sodium nitrite). For example, when the reactive substituted phthalimide comprises a chloro-substituted N-(C₁₋₁₃ alkyl)phthalimide (e.g., 4-chloro-N-methylphthalimide, 3-chloro-N-methylphthalimide, or a combination comprising at least one of the foregoing), the product mixture can further comprise an alkali metal chloride (e.g., sodium chloride). In some embodiments, when present, the inorganic alkali metal salt is present as a solid precipitate in the product mixture (i.e., the inorganic alkali metal salt is not soluble in the product mixture).

The method for producing an aromatic bisimide further comprises introducing the above-described product mixture to a liquid-liquid extraction column under the conditions described below. In some embodiments, the product mixture can be directly transferred to the liquid-liquid extraction column (i.e., no prior purification or washing is necessary). In some embodiments, prior to introducing the product mixture to the liquid-liquid extraction column, water can be added to the product mixture. Thus in some embodiments, the method further comprises separating the product mixture into an aqueous phase and an organic phase comprising the aromatic bisimide, and introducing the organic phase to the liquid-liquid extraction column. The liquid-liquid extraction column is preferably an agitated liquid-liquid extraction column. In some embodiments, the agitation is mechanical agitation. The liquid-liquid extraction can be carried out in a batch-wise or continuous method. In some embodiments, the extracting is a continuous process, i.e., the introducing of the product mixture to the extraction column is continuous. Thus, in some embodiments, the liquid-liquid extraction column is preferably a continuous liquid-liquid extraction column. The temperature of the liquid-liquid extraction column is such that the bisimide product remains dissolved in the solvent at the desired solids content.

The liquid-liquid extraction column includes a 0.01 to 5 weight percent aqueous alkali metal hydroxide solution to extract the dialkali metal salt, the corresponding residual dihydroxy aromatic compound, and the corresponding mono-substituted salt of the dihydroxy aromatic compound by-product from the product mixture. The extraction provides a purified aromatic bisimide, generally as a solution in the nonpolar organic solvent (e.g., toluene). When present, residual substituted phthalimide can also be extracted from the product mixture. The residual substituted phthalimide can be extracted in the form of a water-soluble amide-acid carboxylate salt having the formula 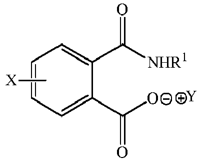 wherein X and R¹ are as described above, and Y is an alkali metal ion, for example, lithium, sodium, potassium, or a combination comprising at least one of the foregoing. In some embodiments, Y is sodium. In some embodiments, X is nitro. In some embodiments, R¹ is methyl. When present, inorganic alkali metal salt (e.g., an alkali metal nitrite) can also be extracted from the product mixture using the liquid-liquid extraction column.

The alkali metal hydroxide can be, for example, sodium hydroxide, or potassium hydroxide, preferably sodium hydroxide. In some embodiments, the liquid-liquid extraction column includes an alkali metal hydroxide aqueous solution having a concentration of 0.05 to 4 weight percent, or 0.1 to 3 weight percent, or 0.1 to 2 weight percent, or 0.5 to 2 weight percent, or 0.5 to 1.5 weight percent. In some embodiments, the liquid-liquid extraction column is operated at a temperature of 70 to 100°C, preferably 75 to 85°C, more preferably 75 to less than 85°C, and a pressure of 0.05 to 20 atm preferably 0.05 to 1.5 atm.

Optionally, the product mixture can be washed with water prior to introducing to the liquid-liquid extraction column. The product mixture can be washed in a batch-wise process, for example at least 1 to 10 times with 0.25 to 10 volumes of water per volume of product mixture to effect the removal of inorganic salts (e.g., alkali metal halide salts, alkali metal nitrite salts, and the like). Alternatively, the product mixture can be washed in a continuous process. Additionally, the purified aromatic bisimide can optionally be washed with water following the extraction process in the liquid-liquid extraction column. The purified aromatic bisimide can be washed in a batch-wise process, for example at least 1 to 10 times with 0.25 to 10 volumes of water per volume of product mixture to effect the removal of inorganic salts. Alternatively, the purified aromatic bisimide can be washed in a continuous process. In some embodiments, washing the product mixture with water prior to introducing to the liquid-liquid extraction column can be at a temperature of 70 to 100°C, preferably 75 to 85°C, more preferably 80 to 85°C, even more preferably, 85°C.

In addition to reacting the dialkali metal salt of the dihydroxy aromatic compound and the reactive substituted phthalimide to form a product mixture, and introducing the product mixture to a liquid-liquid extraction column, the method of the present disclosure further includes recovering from the liquid-liquid extraction column the purified aromatic bisimide having less than 500 parts per million (ppm), or less than 100 ppm, or less than 1 ppm, or less than 10 parts per billion (ppb) of residual dialkali metal salt of the dihydroxy aromatic compound, the corresponding dihydroxy aromatic compound, the corresponding mono-substituted salt of the dihydroxy aromatic compound, or a combination comprising at least one of the foregoing, with respect to the weight of the bisimide product. Stated otherwise, the bisimide contains less than 500 ppm, or less than 100 ppm, or less than 1 ppm, or less than 10 ppb of residual bisphenol A. For example, in some embodiments, the aromatic bisimide can have greater than 0 to less than 500 ppm of residual bisphenol A, or greater than 0 to less than 100 ppm of residual bisphenol A, or greater than 0 to less than 1 ppm of residual bisphenol A, or greater than 0 to less than 10 ppb of residual bisphenol A. In an embodiment, the bisimide preferably contains less than 10 ppb of BPA, for example, greater than 0 to less than 10 ppb of BPA.

The recovering can include, for example, removing the organic solvent to provide the aromatic bisimide.

Another aspect of the present disclosure is a reactor and continuous purification system for the production of an aromatic bisimide. As shown in the Figure, the system includes a first mechanically stirred reaction vessel 10 wherein a dialkali metal salt of the dihydroxy aromatic compound is reacted with a reactive substituted phthalimide in a solvent in the presence of a phase transfer catalyst at the desired temperature to afford a product mixture comprising the aromatic bisimide, solvent, residual amounts of the dialkali metal salt of the dihydroxy aromatic compound and the reactive substituted phthalimide, and catalyst. The product mixture stream (20) is then conveyed to a second mechanically stirred vessel (14) downstream of the first mechanically stirred reaction vessel. The reaction stream (20) is configured to enter the second mechanically stirred vessel (14). The second mechanically stirred vessel thus comprises a product mixture comprising at least one of a dialkali metal salt of a dihydroxy aromatic compound, the corresponding dihydroxy aromatic compound, the corresponding mono-substituted salt of the dihydroxy aromatic compound, or a combination comprising at least one of the foregoing, and an aromatic bisimide. A coalescer (22) is positioned downstream of vessel (14), and is configured to receive the product mixture from a first outlet stream (24) of the vessel (14). The coalescer (22) is further configured to receive a first water inlet stream (34), and wash the product mixture with water in the mixing element of the coalescer. The mixed phases (organic and aqueous) enter the coalescing element within the coalescer to separate the phases. The phases continue to separate in the separation compartment of the coalescer. The organic phase leaves coalescer (22) as a first outlet stream (26) and feeds the extraction column (28). The aqueous phase leaves coalescer (22) in a second outlet stream (36). The water leaving the coalescer (22) contains dissolved inorganic alkali metal salt by-product (e.g., the alkali metal halide or alkali metal nitrite). The system further comprises a liquid-liquid extraction column (28) configured to receive the product mixture from the second outlet stream (26) of the coalescer.

The liquid-liquid extraction column (28) is configured to extract the product mixture with a 0.01 to 5 weight percent aqueous alkali metal hydroxide solution, and is thus configured to receive an aqueous alkali metal hydroxide inlet stream (38) from vessel (50). The phases are mixed in small compartments within column (28). The aqueous phase flows to the bottom of the column and the organic phase, when less dense than the aqueous phase, flows to the top of the column, driven by the density difference of each phase. Extracting the product mixture provides a purified aromatic bisimide stream (30) having less than 10 parts per billion of residual dialkali metal salt of a dihydroxy aromatic compound, the corresponding dihydroxy aromatic compound, the corresponding mono-substituted salt of the dihydroxy aromatic compound, or a combination comprising at least one the foregoing, with respect to the weight of the bisimide product. Water used in the extraction to remove the dialkali metal salt of a dihydroxy aromatic compound, the corresponding dihydroxy aromatic compound, the corresponding mono-substituted salt of the dihydroxy aromatic compound, or a combination comprising at least one the foregoing can be removed from the liquid-liquid extraction column by an aqueous alkali metal hydroxide outlet stream (44). The aqueous alkali metal hydroxide outlet stream (44) can be recycled to vessel (50), configured to receive aqueous alkali metal hydroxide solution (32) and supply the aqueous alkali metal hydroxide inlet stream (38) to the liquid-liquid extraction column (28). A slip stream of stream (44) can be removed from the system to maintain the extraction efficiency of the extraction column. The system can further include a second coalescer (46) downstream of the extraction column (28) configured to receive the purified aromatic bisimide stream (30). The coalescer is further configured to receive a second water inlet stream (40), and wash the product mixture with water, and includes a second water outlet stream (42) to remove water after washing. The second coalescer (46) can provide a second purified aromatic bisimide stream (48) having less than 10 parts per billion of residual dialkali metal salt of a dihydroxy aromatic compound, the corresponding dihydroxy aromatic compound, the corresponding mono-substituted salt of the dihydroxy aromatic compound, or a combination comprising at least one the foregoing with respect to the weight of the bisimide. The purified aromatic bisimide stream 48 can be transferred to a receiving vessel (52).

Another aspect of the present disclosure is a method for the manufacture of a polyetherimide. The method includes contacting an aromatic bisimide prepared according to the above-described method with a phthalic anhydride in the presence of a catalyst to provide an aromatic bis(ether phthalic anhydride) of formula (6) 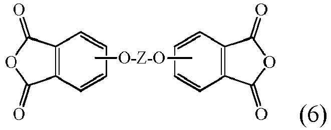 wherein Z is as described in formula (1). The catalyst can be a C₁₋₂₀ trialkylamine, for example trimethylamine. In some embodiments, the contacting can occur in the presence of 0.5 to 15 mole percent of the catalyst with respect to the anhydride. The aromatic bisimide and the phthalic anhydride are contacted under conditions that are generally known to be effective to provide the aromatic bis(ether phthalic anhydride). For example, the phthalic anhydride can present in a molar excess compared to the aromatic bisimide, for example 3 to 8 molar excess of phthalic anhydride relative to aromatic bismide. The contacting can further be in the presence of a solvent, for example water. The contacting can be at a temperature of 150 to 210°C, and can be carried out for 0.5 to 3 hours, preferably with agitation (e.g., stirring).

Polymerization of the aromatic bis(ether phthalic anhydride) (6) with an organic diamine of formula

H₂N-R-NH₂ (7)

provides the polyetherimide. In formula (7) each R is the same or different, and is a substituted or unsubstituted divalent organic group, such as a C₆₋₂₀ aromatic hydrocarbon group, a straight or branched chain C₂₋₂₀ alkylene group, a C₃₋₈ cycloalkylene group, in particular a halogenated derivative of any of the foregoing. In some embodiments R is divalent group of one or more of the following formulas (8) 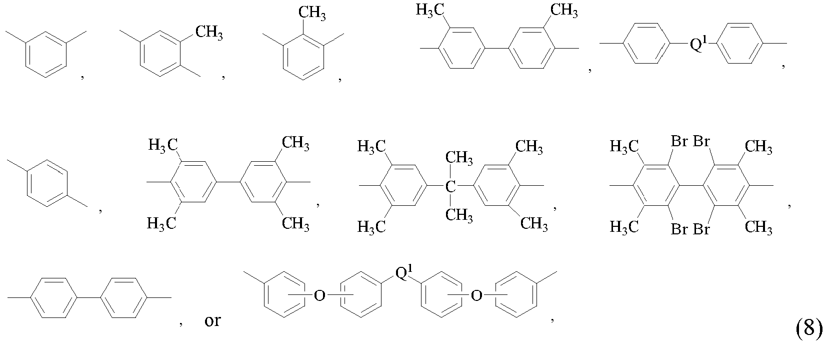 wherein Q¹ is -O-,-S-, -C(O)-, -SO₂-, -SO-, -P(R^{a})(=O)- wherein R^{a} is a C₁₋₈ alkyl or C₆₋₁₂ aryl, -C_{y}H_{2y}- wherein y is an integer from 1 to 5 or a halogenated derivative thereof (which includes perfluoroalkylene groups), or -(C₆H₁₀)_{z}- wherein z is an integer from 1 to 4. In some embodiments R is m-phenylene, p-phenylene, or a diarylene sulfone, in particular bis(4,4'-phenylene)sulfone, bis(3,4'-phenylene)sulfone, bis(3,3'-phenylene)sulfone, or a combination comprising at least one of the foregoing. In some embodiments, at least 10 mole percent of the R groups contain sulfone groups, and in other embodiments no R groups contain sulfone groups. The polyetherimides prepared according to the method disclosed herein advantageously have less than 100 parts per billion, preferably less than 10 ppb of residual bisphenol A.

Another aspect of the present disclosure is a polyetherimide comprising less than 500 ppm, or less than 100 ppm, or less than 1 ppm, or less than 10 ppb, preferably less than 9 ppb of residual bisphenol A. The polyetherimide comprises 2 to 1000, or 5 to 500, or 10 to 100 repeating units of formula (9) 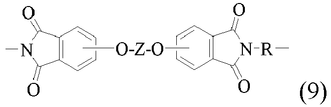 wherein each Z and R is the same or different, and is as described in formulas (1) and (7) above. Further in formula (9), the divalent bonds of the -O-Z-O- group are in the 3,3', 3,4', 4,3', or the 4,4' positions. In an embodiment in formula (9), R is m-phenylene or p-phenylene and Z is a divalent group of formula (2a). Alternatively, R is m-phenylene or p-phenylene and Z is a divalent group of formula (2a) wherein Q is 2,2-isopropylidene. Alternatively, the polyetherimide can be a copolymer comprising additional structural polyetherimide units of formula (9) wherein at least 50 mole percent (mol%) of the R groups are bis(3,4'-phenylene)sulfone, bis(3,3'-phenylene)sulfone, or a combination comprising at least one of the foregoing, and the remaining R groups are p-phenylene, m-phenylene or a combination comprising at least one of the foregoing; and Z is 2,2-(4-phenylene)isopropylidene, i.e., a bisphenol A moiety.

In some embodiments, the polyetherimide is a copolymer that optionally comprises additional structural imide units that are not polyetherimide units, for example imide units of formula (10) 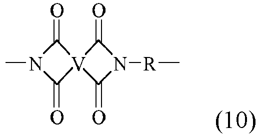 wherein R is as described in formula (7) and each V is the same or different, and is a substituted or unsubstituted C₆₋₂₀ aromatic hydrocarbon group, for example a tetravalent linker of the formulas 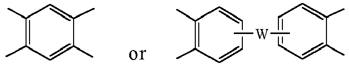 wherein W is a single bond, -O-, -S-, -C(O)-, -SO₂-, -SO-, a C₁₋₁₈ hydrocarbylene group, - P(R^{a})(=O)- wherein R^{a} is a C₁₋₈ alkyl or C₆₋₁₂ aryl, or -C_{y}H_{2y}- wherein y is an integer from 1 to 5 or a halogenated derivative thereof (which includes perfluoroalkylene groups). These additional structural imide units preferably comprise less than 20 mol% of the total number of units, and more preferably can be present in amounts of 0 to 10 mol% of the total number of units, or 0 to 5 mol% of the total number of units, or 0 to 2 mole % of the total number of units. In some embodiments, no additional imide units are present in the polyetherimide.

In some embodiments, the polyetherimide is a polyetherimide-siloxane copolymer including the above-described polyetherimide repeating units and siloxane blocks containing units of formula (12) 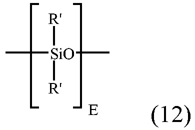 wherein E has an average value of 2 to 100, 2 to 31, 5 to 75, 5 to 60, 5 to 15, or 15 to 40, and each R' is independently a C₁₋₁₃ monovalent hydrocarbyl group. For example, each R' can independently be a C₁₋₁₃ alkyl group, C₁₋₁₃ alkoxy group, C₂₋₁₃ alkenyl group, C₂₋₁₃ alkenyloxy group, C₃₋₆ cycloalkyl group, C₃₋₆ cycloalkoxy group, C₆₋₁₄ aryl group, C₆₋₁₀ aryloxy group, C₇₋₁₃ arylalkyl group, C₇₋₁₃ arylalkoxy group, C₇₋₁₃ alkylaryl group, or C₇₋₁₃ alkylaryloxy group. The foregoing groups can be fully or partially halogenated with fluorine, chlorine, bromine, or iodine, or a combination comprising at least one of the foregoing. In an embodiment no bromine or chlorine is present, and in another embodiment no halogens are present. Combinations of the foregoing R groups can be used in the same copolymer. In an embodiment, the polysiloxane block comprises R' groups that have minimal hydrocarbon content. In a specific embodiment, an R' group with a minimal hydrocarbon content is a methyl group.

The polyetherimide-siloxane copolymers can be formed by polymerization of an aromatic bisanhydride (6) and a diamine component comprising an organic diamine (7) as described above or mixture of diamines, and a polysiloxane diamine of formula (13) 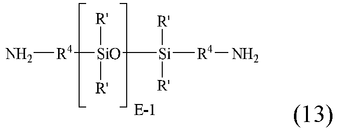 wherein R' and E are as described in formula (12), and each R⁴ is independently a C₂₋C₂₀ hydrocarbon moiety, in particular a C₂₋C₂₀ arylene, alkylene, or arylenealkylene group. In an embodiment R⁴ is a C₂₋C₂₀ alkylene group, specifically a C₂₋C₁₀ alkylene group such as propylene, and E has an average value of 5 to 100, 5 to 75, 5 to 60, 5 to 15, or 15 to 40. Procedures for making the polysiloxane diamines of formula (13) are well known in the art.

In some polyetherimide-siloxane copolymers the diamine component used in the manufacture of the copolymers can contain 10 to 90 mole percent (mol %), or 20 to 50 mol%, or 25 to 40 mol% of polysiloxane diamine (13) and 10 to 90 mol%, or 50 to 80 mol%, or 60 to 75 mol% of diamine (7), for example as described in US Patent 4,404,350. The diamine components can be physically mixed prior to reaction with the bisanhydride(s), thus forming a substantially random copolymer. Alternatively, block or alternating copolymers can be formed by selective reaction of (7) and (13) with aromatic bis(ether anhydrides) (6), to make polyimide blocks that are subsequently reacted together. Thus, the polyetherimide-siloxane copolymer can be a block, random, or graft copolymer. Block polyetherimide-siloxane copolymers comprise etherimide blocks and siloxane blocks in the polymer backbone. The etherimide blocks and the siloxane blocks can be present in random order, as blocks (i.e., AABB), alternating (i.e., ABAB), or a combination thereof. Graft polyetherimide-siloxane copolymers are non-linear copolymers comprising the siloxane blocks connected to linear or branched polymer backbone comprising etherimide blocks.

In an embodiment, the polyetherimide-siloxane copolymer has units of formula (14) 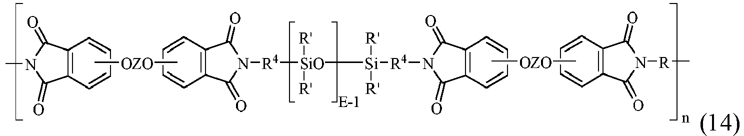 wherein R' and E of the siloxane are as in formula (12), the R and Z of the imide are as in formula (9), R⁴ is the same as R⁴ as in formula (13), and n is an integer from 5 to 100. In a specific embodiment, the R is a phenylene, Z is a residue of bisphenol A, R⁴ is n-propylene, E is 2 to 100, 5 to 30, or 10 to 40, n is 5 to 100, and each R' of the siloxane is methyl.

The relative amount of polysiloxane units and etherimide units in the polyetherimide-siloxane copolymer depends on the desired properties, and are selected using the guidelines provided herein. In particular, the polyetherimide-siloxane copolymer is selected to have a certain average value of E, and is selected and used in amount effective to provide the desired weight percent (wt.%) of siloxane units. In an embodiment the polyetherimide-siloxane comprises 5 to 50 wt.%, 10 to 40 wt.%, or 20 to 35 wt.% siloxane units, based on the total weight of the polyetherimide-siloxane. In some embodiments the polysiloxane block of the copolymer has a number average molecular weight (Mn) of 300 to 3000 grams/mole (Daltons). system for the manufacture of a polyetherimide is also disclosed. The system comprises a reactor comprising a product mixture comprising at least one of a dialkali metal salt of a dihydroxy aromatic compound, the corresponding dihydroxyaromatic compound, or the corresponding mono-substituted salt of the dihydroxy aromatic compound, and an aromatic bisimide of formula (5), as described above.

In some embodiments, the aromatic bisimide is prepared according to the above-described method, and has less than 500 ppm, or less than 100 ppm, or less than 1 ppm, or less than 10 ppb of residual bisphenol A. For example, the aromatic bisimide can have less than 500 ppm, or less than 100 ppm, or less than 1 ppm, or less than 10 ppb of residual dialkali metal salt of the dihydroxy aromatic compound, the corresponding dihydroxy aromatic compound, the corresponding mono-substituted salt of the dihydroxy aromatic compound or a combination comprising at least one of the foregoing. For example, in some embodiments, the aromatic bisimide can have greater than 0 to less than 500 ppm of residual bisphenol A, or greater than 0 to less than 100 ppm of residual bisphenol A, or greater than 0 to less than 1 ppm of residual bisphenol A, or greater than 0 to less than 10 ppb of residual bisphenol A.

An article comprising the polyetherimide having less than 500 ppm, or less than 100 ppm, or less than 1 ppm, or less than 100 parts per billion, or less than 10 ppb of residual dialkali metal salt of the dihydroxy aromatic compound, the corresponding dihydroxy aromatic compound, the corresponding mono-substituted salt of the dihydroxy aromatic compound or a combination comprising at least one of the foregoing, preferably residual bisphenol A, represents another aspect of the present disclosure. Articles including the polyetherimide can be prepared by any number of methods including shaping, foaming, extruding, thermoforming, spinning, or molding. Examples of applications for the articles include food service, medical, lighting, lenses, sight glasses, windows, enclosures, safety shields, cookware, medical devices, trays, plates, handles, helmets, animal cages, electrical connectors, enclosures for electrical equipment, engine parts, automotive engine parts, lighting sockets and reflectors, electric motor parts, power distribution equipment, communication equipment, computers, and the like. Articles can include, for example, hollow fibers, hollow tubes, hollow tube fibers wherein the wall of the fiber has small openings of various pore sizes which affords a permeable membrane fiber, permeable membranes in other shapes with various pore sizes, solid fibers, sheets, films, multilayer sheets, multilayer films, molded parts, extruded profiles, coated parts, foams, windows, luggage racks, wall panels, chair parts, lighting panels, diffusers, shades, partitions, lenses, skylights, lighting devices, reflectors, ductwork, cable trays, conduits, pipes, cable ties, wire coatings, electrical connectors, air handling devices, ventilators, louvers, insulation, bins, storage containers, doors, hinges, handles, sinks, mirror housing, mirrors, toilet seats, hangers, coat hooks, shelving, ladders, hand rails, steps, carts, trays, cookware, food service equipment, medical devices, data transmission equipment, powders, composites, communications equipment and instrument panels, and the like.

The method for producing aromatic bismides described herein advantageously allows for the production of aromatic bismides having less than 500 ppm, or less than 100 ppm, or less than 1 ppm, or less than 10 ppb of a residual dihydroxy aromatic compound (e.g., bisphenol A). Accordingly, polyetherimides can now be produced having less than 100 parts per billion, or less than 10 ppb of a residual dihydroxy aromatic compound (e.g., bisphenol A). The polyetherimides meet new customer requirements for articles for various applications. Therefore, a substantial improvement in methods for producing aromatic bisimides and polyetherimides is provided.

The methods and systems for the manufacture of aromatic bisimides and polyetherimides are further illustrated by the following non-limiting examples.

### EXAMPLES

### Examples 1-4

An aromatic bisimide was prepared according to the method disclosed herein.

A disodium salt of bisphenol A (100 grams, 367 millimole (mmol)) was reacted with nitrophthalimide comprising 3-nitrophthalimide and 4-nitrophthalimide (151.47 grams, 735 mmol) in toluene (640 milliliters) in the presence of hexaethylguanidinium chloride (HEGCl; 1.0 grams, 3.7 mmol) as a phase transfer catalyst. The reaction was carried out at 120°C for 2 hours with stirring under nitrogen.

The product mixture was cooled to 83°C and mixed with 200 milliliters of water at 83°C. The mixture was stirred for 5 minutes at 83°C. The stirring was stopped and the aqueous solution phase was allowed to separate from the organic solution phase containing the bisimide over a 10 minute period. The aqueous phase was separated from the organic phase.

The resulting organic phase was purified using a mechanically agitated, continuous liquid-liquid extraction column at 80°C. The reaction mixture was about 25 wt.% aromatic bisimide in toluene with residual concentrations of various starting materials including the disodium and monosodium salt of bisphenol A, bisphenol A, the corresponding mono-substituted imide, and nitrophthalimide. The reaction/purification procedure described was scaled up to provide enough material for extraction studies.

A 1 wt.% aqueous sodium hydroxide solution at 80 °C and the organic product (aromatic bisimide in toluene having been once extracted with water, as described above) at 80 °C were introduced to the extraction column to extract the residual starting materials. The extraction column was three inches in diameter, and was mechanically agitated at 400 rpm, with the agitator diameter being 35% of the column diameter. The extraction column included 99 agitated stages, each having a height of 1 inch.

Purification using the liquid-liquid extraction procedure described above provided the aromatic bisimide product having a non-detectable level of bisphenol A (i.e., less than 10 ppb bisphenol A with respect to the dry weight of the bisimide), as shown in Table 1.

**Table 1**

| Ex. | Feed Rate (organic phase; g/min) | 1 wt% NaOH(aq) Feed Rate (g/min) | RPM | # stages | BPA¹ (ppb) |
|---|---|---|---|---|---|
| E1 | 240 | 120 | 350 | 99 | -- |
| E2 | 240 | 120 | 400 | 99 | -- |
| E3 | 185 | 37 | 550 | 81 | 13 |
| E4 | 174 | 52 | 550 | 81 | 202 |

| | | | | | |
|---|---|---|---|---|---|
| ¹Residual BPA detected in aromatic bisimide product | | | | | |

As demonstrated by Examples 1-4 shown in Table 1, residual bisphenol A can be reduced to non-detectable levels (i.e., less than 10 ppb based) in an aromatic bisimide using the method described herein.

### Comparative Examples 1-4

As a comparative example, removal of residual bisphenol A was attempted under vacuum at elevated temperature in a simulated wiped film evaporator. A solid mixture of 4,4'-bisphenol-A-bis-N-methylphthalimide, 3,4'-bisphenol-A-bis-N-methylphthalimide, and 3,3'-bisphenol-A-bis-N-methylphthalimide containing 3.9 ppm of residual BPA (5 grams) was charged to a 250-mL, round-bottomed flask. The flask was then placed in an oven and connected to a vacuum pump using a triple-bulb Kuglerohr adapter. The flask was oscillated 180°C with an air driven motor. The oven was rapidly heated to 280°C and the flask was rotated from 10 to 30 minutes under different levels of vacuum. Under these conditions, the bisimide melted and formed a thin film on the inside of the flask. After a prescribed amount of time, nitrogen was allowed to enter the flask and the vacuum was broken. The flask removed from the oven and the bisimide allowed to cool. The resulting bisimide was then analyzed for residual bisimide by high pressure liquid chromatograph (HPLC). The results are shown in Table 2.

**Table 2**

| Ex. | Temperature (°C) | Time (mins) | Vacuum (mm) | Residual BPA (ppm) |
|---|---|---|---|---|
| Starting BI | | | | 3.9 |
| CE1 | 280 | 10 | 0.7 | 0.5 |
| CE2 | 280 | 15 | 0.08 | 0.7 |
| CE3 | 280 | 20 | 0.01 | 0.7 |
| CE4 | 280 | 30 | 0.01 | 1.0 |

As shown in Table 2, although some BPA was removed under vacuum at elevated temperature, the desired low level of BPA was not achieved.

### Comparative Example 5

As a further comparative example, removal of residual bisphenol A was attempted from bisimide/toluene mixtures in a laboratory simulated series of batch extractors. A 2-liter, oil-jacketed vessel with a bottom drain valve, equipped with a mechanical agitator that rotated a paddle blade stirrer, was charge with 800 grams of a reaction mixture containing toluene, bisimide (composed of 4,4'-bisphenol-A-bis-N-methylphthalimide, 3,4'-bisphenol-A-bis-N-methylphthalimide, and 3,3'-bisphenol-A-bis-N-methylphthalimide), sodium alkali metal, residual BPA disodium salt, and other impurities. The material was heated to 85°C and agitated under nitrogen. The bisimide dissolved in the toluene to afford a 24 weight percent (wt%) solution. The reaction mixture was then agitated with 100 mL 1 wt% of aqueous sodium hydroxide for 5 minutes at 83 to 85°C. The agitation was such that there was turbulent mixing of the organic and aqueous phases. The agitation was stopped and the phases were allowed to separate at 85°C for 10 minutes. The aqueous phase was drained from the bottom of the vessel. The organic phase was then analyzed for residual BPA using HPLC. The toluene phase was washed 3 more times as described above, and after each wash the amount of residual BPA present in the bisimide/toluene phase was determined by HPLC. The results are shown in Table 3.

**Table 3**

| Wash # | Temperature (°C) | Residual BPA in BI (ppm) |
|---|---|---|
| 1 | 85 | NM¹ |
| 2 | 85 | NM¹ |
| 3 | 85 | 0.094 |
| 4 | 85 | 0.099 |
| 5 | 85 | 0.103 |

| | | |
|---|---|---|
| "NM" means "not measured" | | |

The experiment showed that BPA can be washed out of the bisimide/toluene solution with 5 multiple caustic batch washes to a level of about 100 ppb (i.e., 100 ppb of BPA with respect to the dry weight of bisimide).

### Comparative Examples 6-9

Repeated batchwise extractive purification of a bisimide/toluene solution was carried out according to procedure described above for comparative example 5. 800 grams of reaction mixture was extracted twice with 100 mL of 1 wt% NaOH (aqueous). The purified bisimide/toluene solution was then washed three additional times with 1 wt% NaOH in the same manner as described above. The amount of BPA remaining in the bisimide was determined by HPLC after each wash. The experiment was done four times and the data appears in Table 4.

**Table 4**

| | CE6 | CE7 | CE8 | CE9 |
|---|---|---|---|---|
| Initial Level of BPA in BI (ppb) | 5400 | 11400 | 11300 | 1900 |
| Wt% BI in toluene | 22.6 | 22.8 | 22.7 | 22.7 |
| 1^{st} additional wash (ppb) | 108 | 211 | 187 | 376 |
| 2^{nd} additional wash (ppb) | 30 | 46 | 37 | 44 |
| 3^{rd} additional wash (ppb) | 34 | 24 | 19 | 27 |

As shown in Table 4, using a repeated batch extractive purification process, the residual amount of BPA in the aromatic bisimide was reduced from 5.4-19 ppm to 19-34 ppb.

### Comparative Examples 10-11

A 5-liter, oil-jacketed vessel with a bottom drain valve, equipped with a mechanical agitator that rotated a paddle blade stirrer, was charged with 2400 grams of a reaction mixture containing toluene, bisimide (composed of 4,4'-bisphenol-A-bis-N-methylphthalimide, 3,4'-bisphenol-A-bis-N-methylphthalimide, and 3,3'-bisphenol-A-bis-N-methylphthalimide), sodium alkali metal, residual BPA disodium salt, and other impurities. The material was heated to 85°C and agitated under nitrogen. The bisimide dissolved in the toluene to afford a 24 weight percent (wt%) solution. The reaction mixture was then agitated with 100 mL 1 wt% of aqueous sodium hydroxide for 5 minutes at 83 to 85°C. The agitation was such that there was turbulent mixing of the organic and aqueous phases. The agitation was stopped and the phases were allowed to separate at 85°C for 10 minutes. The aqueous phase was drained from the bottom of the vessel. The toluene phase was washed again as described above. The extractively purified toluene/bisimide phase was then charged to a separate vessel and the toluene distilled overhead. The bisimide remaining in the vessel was then heated to 250°C under nitrogen to remove the last traces of toluene to afford neat bisimide.

The neat bisimide (200 grams), comprised of a mixture of 4,4'-bisphenol-A-bis-N-methylphthalimide, 3,4'-bisphenol-A-bis-N-methylphthalimide, and 3,3'-bisphenol-A-bis-N-methylphthalimide, was dissolved in toluene at 85°C in a 2-liter, oil-jacketed vessel with a bottom drain valve, equipped with a mechanical agitator that rotated a paddle blade stirrer. Two trials were run, one at 17.2 wt% bisimide in toluene, and one at 13.9 wt%. The toluene/bisimide phase as washed three times with 100 mL of aqueous 1 wt% sodium hydroxide as described above, and a sample of the bisimide after each wash was analyzed for residual BPA. The results are shown in Table 5.

**Table 5**

| | CE10 | CE11 |
|---|---|---|
| Initial Level of BPA in BI (ppb) | 5900 | 6800 |
| Wt% BI in toluene | 17.2 | 13.9 |
| 1^{st} additional wash (ppb) | 108 | 116 |
| 2^{nd} additional wash (ppb) | 31 | 36 |
| 3^{rd} additional wash (ppb) | 27 | 33 |

As shown in Table 5, the level of the residual BPA in the bisimide obtained after washing was 27 to 33 ppb.

The methods and systems for the manufacture of aromatic bisimides and polyetherimides are further illustrated by the following embodiments.

Embodiment 1: A method for producing an aromatic bisimide, the method comprising reacting a dialkali metal salt of a dihydroxy aromatic compound with a reactive substituted phthalimide under conditions effective to form a product mixture comprising at least one of the dialkali metal salt, the corresponding dihydroxy aromatic compound, the corresponding mono-substituted salt of the dihydroxy aromatic compound, or a combination comprising at least one of the foregoing, and the aromatic bisimide; introducing the product mixture to an agitated liquid-liquid extraction column with a 0.01 to 5 weight percent aqueous alkali metal hydroxide solution to extract residual dialkali metal salt, the corresponding dihydroxy aromatic compound, and the corresponding mono-substituted salt of the dihydroxy aromatic compound from the product mixture; and recovering from the liquid-liquid extraction column a purified aromatic bisimide having less than 500 ppm, or less than 100 ppm, or less than 1 ppm, or less than 10 parts per billion of residual dialkali metal salt, the corresponding dihydroxy aromatic compound, the corresponding mono-substituted salt of the dihydroxy aromatic compound or a combination comprising at least one of the foregoing.

The terms "a" and "an" and "the" as used herein do not denote a limitation of quantity, and are to be construed to cover both the singular and the plural, unless otherwise indicated herein or clearly contradicted by context. "Or" means "and/or" unless clearly indicated otherwise. All ranges disclosed herein are inclusive of the endpoints, and the endpoints are independently combinable with each other. "Combination" is inclusive of blends, mixtures, alloys, reaction products, and the like.

As used herein, the term "hydrocarbyl" includes groups containing carbon, hydrogen, and optionally one or more heteroatoms (e.g., 1, 2, 3, or 4 atoms such as halogen, O, N, S, P, or Si). "Alkyl" means a branched or straight chain, saturated, monovalent hydrocarbon group, e.g., methyl, ethyl, i-propyl, and n-butyl. "Alkylene" means a straight or branched chain, saturated, divalent hydrocarbon group (e.g., methylene (-CH₂-) or propylene (-(CH₂)₃-)). "Alkenyl" and "alkenylene" mean a monovalent or divalent, respectively, straight or branched chain hydrocarbon group having at least one carbon-carbon double bond (e.g., ethenyl (-HC=CH₂) or propenylene (-HC(CH₃)=CH₂-). "Alkynyl" means a straight or branched chain, monovalent hydrocarbon group having at least one carbon-carbon triple bond (e.g., ethynyl). "Alkoxy" means an alkyl group linked via an oxygen (i.e., alkyl-O-), for example methoxy, ethoxy, and sec-butyloxy. "Cycloalkyl" and "cycloalkylene" mean a monovalent and divalent cyclic hydrocarbon group, respectively, of the formula -CₙH₂ₙ₋ₓ and -CₙH₂ₙ₋₂ₓ- wherein x is the number of cyclization(s). "Aryl" means a monovalent, monocyclic, or polycyclic aromatic group (e.g., phenyl or naphthyl). "Arylene" means a divalent, monocyclic, or polycyclic aromatic group (e.g., phenylene or naphthylene). The prefix "halo" means a group or compound including one more halogen (F, Cl, Br, or I) substituents, which can be the same or different. The prefix "hetero" means a group or compound that includes at least one ring member that is a heteroatom (e.g., 1, 2, or 3 heteroatoms, wherein each heteroatom is independently N, O, S, or P.

"Substituted" means that the compound or group is substituted with at least one (e.g., 1, 2, 3, or 4) substituents instead of hydrogen, where each substituent is independently nitro (-NO₂), cyano (-CN), hydroxy (-OH), halogen, thiol (-SH), thiocyano (-SCN), C₁₋₆ alkyl, C₂₋₆ alkenyl, C₂₋₆ alkynyl, C₁₋₆ haloalkyl, C₁₋₉ alkoxy, C₁₋₆ haloalkoxy, C₃₋₁₂ cycloalkyl, C₅₋₁₈ cycloalkenyl, C₆₋₁₂ aryl, C₇₋₁₃ arylalkylene (e.g, benzyl), C₇₋₁₂ alkylarylene (e.g, toluyl), C₄₋₁₂ heterocycloalkyl, C₃₋₁₂ heteroaryl, C₁₋₆ alkyl sulfonyl (-S(=O)₂-alkyl), C₆₋₁₂ arylsulfonyl (-S(=O)₂-aryl), or tosyl (CH₃C₆H₄SO₂-), provided that the substituted atom's normal valence is not exceeded, and that the substitution does not significantly adversely affect the manufacture, stability, or desired property of the compound. When a compound is substituted, the indicated number of carbon atoms is the total number of carbon atoms in the group, including those of the substituent(s).

## Claims

1. A method for producing an aromatic bisimide, the method comprising
reacting a dialkali metal salt of a dihydroxy aromatic compound of the formula
M⁺⁻O-Z-O⁻⁺M
with a reactive substituted phthalimide of the formula 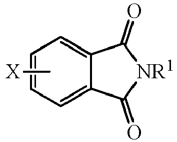 under conditions effective to form a product mixture comprising at least one of the dialkali metal salt of the dihydroxy aromatic compound, a dihydroxy aromatic compound corresponding to the dialkali metal salt of the dihydroxy aromatic compound, a mono-substituted salt of the dihydroxy aromatic compound of the formula 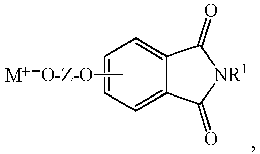 or a combination comprising at least one of the foregoing, and the aromatic bisimide, wherein the aromatic bisimide is of the formula 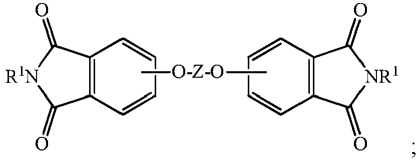
introducing the product mixture to an agitated liquid-liquid extraction column with a 0.01 to 5 weight percent aqueous alkali metal hydroxide solution to extract residual dialkali metal salt, the corresponding dihydroxy aromatic compound, and the corresponding mono-substituted salt of the dihydroxy aromatic compound from the product mixture; and
recovering from the liquid-liquid extraction column a purified aromatic bisimide having less than 500 ppm, or less than 100 ppm, or less than 1 ppm, or less than 10 parts per billion of residual dialkali metal salt, the corresponding dihydroxy aromatic compound, the corresponding mono-substituted salt of the dihydroxy aromatic compound or a combination comprising at least one of the foregoing;
wherein in the foregoing formulas,
M is lithium, sodium, or potassium;
Z is a divalent group of the formula 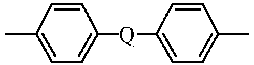
wherein Q is -CyH2y- wherein y is an integer from 1 to 5 or a halogenated derivative thereof,
X is nitro, and
R¹ is a monovalent C₁₋₄ alkyl group.

2. The method of claim 1, further comprising, prior to introducing the product mixture to the liquid-liquid extraction column, adding water to the product mixture and separating the product mixture into an aqueous phase and an organic phase comprising the aromatic bisimide, and introducing the organic phase to the liquid-liquid extraction column.

3. The method of claim 1 or 2, wherein the product mixture comprises the dialkali metal salt of the dihydroxy aromatic compound, the corresponding dihydroxy aromatic compound, the corresponding mono-substituted salt of the dihydroxy aromatic compound, the aromatic bisimide, and, optionally, an alkali metal salt, preferably an alkali metal nitrite, an alkali metal halide, or a combination comprising at least one of the foregoing.

4. The method of any one or more of claims 1 to 3, wherein the liquid-liquid extraction column is a continuous liquid-liquid extraction column.

5. The method of any one or more of claims 1 to 4, wherein the liquid-liquid extraction column is operated at a temperature of 70 to 100°C, preferably 75 to 85°C, and a pressure of 0.05 to 20 atm (0.005 to 2.0265 MPa), preferably 0.05 to 1.5 atm (0.005 to 0.151988 MPa).

6. The method of claim 1, wherein
M is sodium;
Z is 2,2-(4-phenylene)isopropylidene; and
X is nitro and R¹ is a methyl.

7. The method of any one or more of claims 1 to 6, wherein the reactive substituted phthalimide comprises 4-nitro-N-methylphthalimide, 3-nitro-N-methylphthalimide, or a combination comprising at least one of the foregoing.

8. The method of any one or more of claims 1 to 7, wherein the aromatic bisimide comprises 4,4'-bisphenol-A-bis-N-methylphthalimide, 3,4'-bisphenol-A-bis-N-methylphthalimide, 3,3'-bisphenol-A-bis-N-methylphthalimide, or a combination comprising at least one of the foregoing.

9. The method of any one or more of claims 1 to 8, wherein the product mixture further comprises at least one of
residual reactive substituted phthalimide or a derivative thereof;
a nonpolar organic solvent, preferably wherein the nonpolar organic solvent is toluene; or
a phase transfer catalyst, preferably wherein the phase transfer catalyst is a hexaalkylguanidinium salt, more preferably wherein the phase transfer catalyst is hexaethylguanidinium chloride.

10. A method for the manufacture of a polyetherimide, comprising
contacting an aromatic bisimide prepared by the method of any one or more of claims 1 to 9, with a phthalic anhydride in the presence of a catalyst and under conditions effective to provide an aromatic bis(ether phthalic anhydride) of the formula 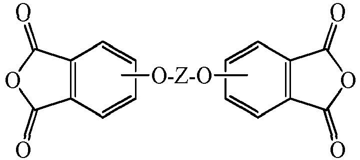
wherein Z is an aromatic C₆₋₂₄ monocyclic or polycyclic moiety optionally substituted with 1 to 6 C₁₋₈ alkyl groups, 1 to 8 halogen atoms, or a combination comprising at least one of the foregoing; and
contacting the aromatic bis(ether phthalic anhydride) with an organic diamine of the formula
H₂N-R²-NH₂
wherein R² is an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 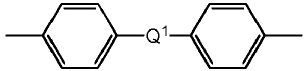
wherein Q¹ is -C_{y}H_{2y}- wherein y is an integer from 1 to 5, and
wherein the polyetherimide has less than 10 parts per billion, preferably less than 9 parts per billion of residual dialkali metal salt of a dihydroxy aromatic compound, the corresponding dihydroxy aromatic compound, the corresponding mono-substituted salt of the dihydroxy aromatic compound or a combination comprising at least one of the foregoing.

## Patentansprüche

1. Verfahren zur Herstellung eines aromatischen Bisimids, wobei das Verfahren Folgendes umfasst:
Umsetzen eines Dialkalimetallsalzes einer aromatischen Dihydroxyverbindung der Formel
M⁺⁻O-Z-O⁻⁺M
mit einem reaktiven substituierten Phthalimid der Formel 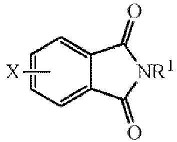 unter wirksamen Bedingungen zur Bildung eines Produktgemischs, das mindestens eines von dem Dialkalimetallsalz der aromatischen Dihydroxyverbindung, einer dem Dialkalimetallsalz der aromatischen Dihydroxyverbindung entsprechenden aromatischen Dihydroxyverbindung, einem monosubstituierten Salz der aromatischen Dihydroxyverbindung der Formel 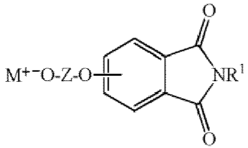 oder einer mindestens eines der Vorangehenden umfassenden Kombination und das aromatische Bisimid umfasst,
wobei das aromatische Bisimid die Formel 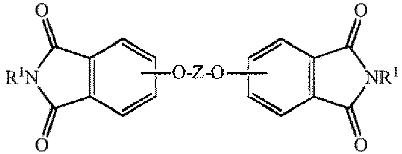 aufweist;
Einbringen des Produktgemischs in eine gerührte Flüssig-Flüssig-Extraktionskolonne mit einer 0,01- bis 5-gewichtsprozentigen wässrigen Alkalimetallhydroxidlösung, um verbleibendes Dialkalimetallsalz, die entsprechende aromatische Dihydroxyverbindung und das entsprechende monosubstituierte Salz der aromatischen Dihydroxyverbindung aus dem Produktgemisch zu extrahieren; und
Rückgewinnen, aus der Flüssig-Flüssig-Extraktionskolonne, eines gereinigten aromatischen Bisimids mit weniger als 500 ppm oder weniger als 100 ppm oder weniger als 1 ppm oder weniger als 10 Teilen pro Milliarde des verbleibenden Dialkalimetallsalzes, der entsprechenden aromatischen Dihydroxyverbindung, dem entsprechenden monosubstituierten Salz der aromatischen Dihydroxyverbindung oder einer mindestens eines der Vorangehenden umfassenden Kombination;
wobei in den vorangehenden Formeln
M für Lithium, Natrium oder Kalium steht;
Z für eine zweiwertige Gruppe der Formel 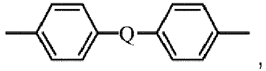
wobei Q für -CyH2y-, wobei y eine ganze Zahl von 1 bis 5 ist, steht, oder ein halogeniertes Derivat davon steht,
X für Nitro steht und
R¹ für eine einwertige C₁₋₄-Alkylgruppe steht.

2. Verfahren nach Anspruch 1, ferner umfassend vor dem Einbringen des Produktgemischs in die Flüssig-Flüssig-Extraktionskolonne das Zusetzen von Wasser zu dem Produktgemisch, Trennen des Produktgemischs in eine wässrige Phase und eine das aromatische Bisimid umfassende organische Phase und Einbringen der organischen Phase in die Flüssig-Flüssig-Extraktionskolonne.

3. Verfahren nach Anspruch 1 oder 2, wobei das Produktgemisch das Dialkalimetallsalz der aromatischen Dihydroxyverbindung, die entsprechende aromatische Dihydroxyverbindung, das entsprechende monosubstituierte Salz der aromatischen Dihydroxyverbindung, das aromatische Bisimid und gegebenenfalls ein Alkalimetallsalz, vorzugsweise ein Alkalimetallnitrit, ein Alkalimetallhalogenid, oder eine mindestens eines der Vorangehenden umfassende Kombination umfasst.

4. Verfahren nach irgendeinem oder mehreren der Ansprüche 1 bis 3, wobei die Flüssig-Flüssig-Extraktionskolonne eine kontinuierliche Flüssig-Flüssig-Extraktionskolonne ist.

5. Verfahren nach irgendeinem oder mehreren der Ansprüche 1 bis 4, wobei die Flüssig-Flüssig-Extraktionskolonne bei einer Temperatur von 70 bis 100 °C, vorzugsweise 75 bis 85 °C, und einem Druck von 0,05 bis 20 atm (0,005 bis 2,0265 MPa), vorzugsweise 0,05 bis 1,5 atm (0,005 bis 0,151988 MPa) betrieben wird.

6. Verfahren nach Anspruch 1, wobei
M für Natrium steht;
Z für 2,2-(4-Phenylen)isopropyliden steht; und
X für Nitro steht und R¹ für Methyl steht.

7. Verfahren nach irgendeinem oder mehreren der Ansprüche 1 bis 6, wobei das reaktive substituierte Phthalimid 4-Nitro-N-methylphthalimid, 3-Nitro-N-methylphthalimid oder eine mindestens eines der Vorangehenden umfassende Kombination umfasst.

8. Verfahren nach irgendeinem oder mehreren der Ansprüche 1 bis 7, wobei das aromatische Bisimid 4,4'-Bisphenol-A-bis-N-methylphthalimid, 3,4'-Bisphenol-A-bis-N-methylphthalimid, 3,3'-Bisphenol-A-bis-N-methylphthalimid oder eine mindestens eines der Vorangehenden umfassende Kombination umfasst.

9. Verfahren nach irgendeinem oder mehreren der Ansprüche 1 bis 8, wobei das Produktgemisch ferner mindestens eines von Folgendem umfasst:
verbleibendes reaktives substituiertes Phthalimid oder ein Derivat davon;
ein unpolares organisches Lösungsmittel, wobei das unpolare organische Lösungsmittel vorzugsweise Toluol ist; oder
einen Phasentransferkatalysator, wobei der Phasentransferkatalysator vorzugsweise ein Hexaalkylguanidiniumsalz ist, wobei der Phasentransferkatalysator bevorzugter Hexaethylguanidiniumchlorid ist.

10. Verfahren zur Herstellung eines Polyetherimids, umfassend:
Inkontaktbringen eines durch das Verfahren nach irgendeinem oder mehreren der Ansprüche 1 bis 9 hergestellten aromatischen Bisimids mit einem Phthalsäureanhydrid in Gegenwart eines Katalysators und unter wirksamen Bedingungen zur Bildung eines aromatischen Bis(etherphthalsäureanhydrids) der Formel 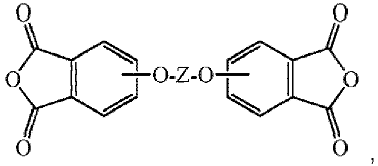
wobei Z für einen aromatischen monocyclischen oder polycyclischen C₆-₂₄-Rest steht, der gegebenenfalls mit 1 bis 6 C₁₋₈-Alkylgruppen, 1 bis 8 Halogenatomen oder einer mindestens eines der Vorangehenden umfassenden Kombination substituiert ist; und
Inkontaktbringen des aromatischen Bis(etherphthalsäureanhydrids) mit einem organischen Diamin der Formel
H₂N-R²-NH₂,
wobei R² für Folgendes steht: eine aromatische Kohlenwasserstoffgruppe mit 6 bis 27 Kohlenstoffatomen, ein halogeniertes Derivat davon, eine gerad- oder verzweigtkettige Alkylengruppe mit 2 bis 10 Kohlenstoffatomen, ein halogeniertes Derivat davon, eine Cycloalkylengruppe mit 3 bis 20 Kohlenstoffatomen, ein halogeniertes Derivat davon, -(C₆H₁₀)_{z}-, wobei z eine ganze Zahl von 1 bis 4 ist, einen aromatischen Hydrocarbylrest mit 1 bis 6 aromatischen Gruppen und eine zweiwertige Gruppe der Formel 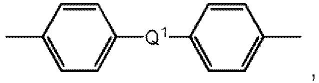
wobei Q¹ für -C_{y}H_{2y}-, wobei y eine ganze Zahl von 1 bis 5 ist, steht, und
wobei das Polyetherimid weniger als 10 Teile pro Milliarde, vorzugsweise weniger als 9 Teile pro Milliarde des verbleibenden Dialkalimetallsalzes einer aromatischen Dihydroxyverbindung, der entsprechenden aromatischen Dihydroxyverbindung, des entsprechenden monosubstituierten Salzes der aromatischen Dihydroxyverbindung oder einer mindestens eines der Vorangehenden umfassenden Kombination aufweist.

## Revendications

1. Procédé de production d'un bisimide aromatique, le procédé comprenant
la réaction d'un sel de métal dialcalin d'un composé aromatique à fonction dihydroxy de formule
M⁺⁻O-Z-O⁻⁺M
avec un phtalimide substitué réactif de formule 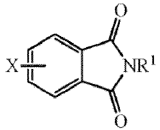 dans des conditions efficaces pour former un mélange de produits comprenant au moins l'un du sel de métal dialcalin du composé aromatique à fonction dihydroxy, d'un composé aromatique à fonction dihydroxy correspondant au sel de métal dialcalin du composé aromatique à fonction dihydroxy, d'un sel monosubstitué du composé aromatique à fonction dihydroxy de formule 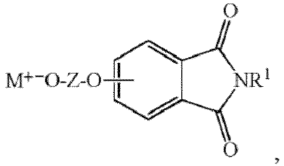 ou d'une combinaison comprenant au moins l'un des composés susmentionnés, et le bisimide aromatique, dans lequel le bisimide aromatique répond à la formule 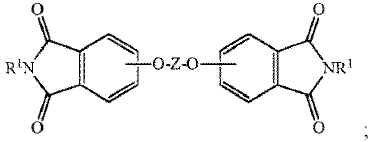
l'introduction du mélange de produits dans une colonne d'extraction liquide-liquide sous agitation avec une solution aqueuse d'hydroxyde de métal alcalin à 0,01 à 5 pour cent en poids pour extraire le sel de métal dialcalin résiduel, le composé aromatique à fonction dihydroxy correspondant et le sel monosubstitué correspondant du composé aromatique à fonction dihydroxy à partir du mélange de produits ; et
la récupération à partir de la colonne d'extraction liquide-liquide d'un bisimide aromatique purifié ayant moins de 500 ppm, ou moins de 100 ppm, ou moins de 1 ppm, ou moins de 10 parties par milliard de sel de métal dialcalin résiduel, du composé aromatique à fonction dihydroxy correspondant, du sel monosubstitué correspondant du composé aromatique à fonction dihydroxy ou d'une combinaison comprenant au moins l'un des composés susmentionnés ;
dans lequel dans les formules précédentes,
M est le lithium, le sodium ou le potassium ;
Z est un groupe divalent de formule 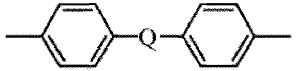
dans laquelle Q est -CyH2y- dans lequel y est un nombre entier de 1 à 5 ou un dérivé halogéné de celui-ci,
X est un nitro, et
R¹ est un groupe alkyle en C₁₋₄ monovalent.

2. Procédé selon la revendication 1, comprenant en outre, avant l'introduction du mélange de produits dans la colonne d'extraction liquide-liquide, l'ajout d'eau au mélange de produits et la séparation du mélange de produits en une phase aqueuse et une phase organique comprenant le bisimide aromatique, et l'introduction de la phase organique dans la colonne d'extraction liquide-liquide.

3. Procédé selon la revendication 1 ou 2, dans lequel le mélange de produits comprend le sel de métal dialcalin du composé aromatique à fonction dihydroxy, le composé aromatique à fonction dihydroxy correspondant, le sel monosubstitué correspondant du composé aromatique à fonction dihydroxy, le bisimide aromatique et, éventuellement, un sel de métal alcalin, de préférence un nitrite de métal alcalin, un halogénure de métal alcalin, ou une combinaison comprenant au moins l'un des composés susmentionnés.

4. Procédé selon l'une quelconque ou plusieurs des revendications 1 à 3, dans lequel la colonne d'extraction liquide-liquide est une colonne d'extraction liquide-liquide continue.

5. Procédé selon l'une quelconque ou plusieurs des revendications 1 à 4, dans lequel la colonne d'extraction liquide-liquide fonctionne à une température de 70 à 100 °C, de préférence de 75 à 85 °C, et à une pression de 0,05 à 20 atm (0,005 à 2,0265 MPa), de préférence de 0,05 à 1,5 atm (0,005 à 0,151988 MPa).

6. Procédé selon la revendication 1, dans lequel
M est le sodium ;
Z est le 2,2-(4-phénylène)isopropylidène ; et
X est un nitro et R¹ est un méthyle.

7. Procédé selon l'une quelconque ou plusieurs des revendications 1 à 6, dans lequel le phtalimide substitué réactif comprend le 4-nitro-N-méthylphtalimide, le 3-nitro-N-méthylphtalimide, ou une combinaison comprenant au moins l'un des composés susmentionnés.

8. Procédé selon l'une quelconque ou plusieurs des revendications 1 à 7, dans lequel le bisimide aromatique comprend le 4,4'-bisphénol-A-bis-N-méthylphtalimide, le 3,4'-bisphénol-A-bis-N-méthylphtalimide, le 3,3'-bisphénol-A-bis-N-méthylphtalimide, ou une combinaison comprenant au moins l'un des composés susmentionnés.

9. Procédé selon l'une quelconque ou plusieurs des revendications 1 à 8, dans lequel le mélange de produits comprend en outre au moins un parmi
le phtalimide substitué réactif résiduel ou un dérivé de celui-ci ;
un solvant organique non polaire, de préférence dans lequel le solvant organique non polaire est le toluène ; ou
un catalyseur de transfert de phase, de préférence dans lequel le catalyseur de transfert de phase est un sel d'hexaalkylguanidinium, plus préférablement dans lequel le catalyseur de transfert de phase est le chlorure d'hexaéthylguanidinium.

10. Procédé de fabrication d'un polyétherimide, comprenant
la mise en contact d'un bisimide aromatique préparé par le procédé selon l'une quelconque ou plusieurs des revendications 1 à 9, avec un anhydride phtalique en présence d'un catalyseur et dans des conditions efficaces pour fournir un bis(anhydride d'éther phtalique) aromatique de formule 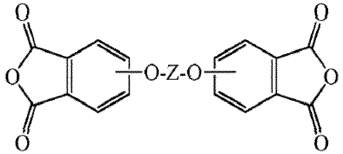
dans laquelle Z est une fraction monocyclique ou polycyclique aromatique en C₆₋₂₄ éventuellement substituée par 1 à 6 groupes alkyle en C₁₋₈, 1 à 8 atomes d'halogène, ou une combinaison comprenant au moins l'un des éléments susmentionnés ; et
la mise en contact du bis(anhydride d'éther phtalique) aromatique avec une diamine organique de formule
H₂N-R²-NH₂
dans laquelle R² est un groupe hydrocarboné aromatique ayant 6 à 27 atomes de carbone, un dérivé halogéné de celui-ci, un groupe alkylène à chaîne droite ou ramifiée ayant 2 à 10 atomes de carbone, un dérivé halogéné de celui-ci, un groupe cycloalkylène ayant 3 à 20 atomes de carbone, un dérivé halogéné de celui-ci, -(C₆H₁₀)_{z}- dans lequel z est un nombre entier de 1 à 4, une fraction hydrocarbyle aromatique ayant de 1 à 6 groupes aromatiques, et un groupe divalent de formule 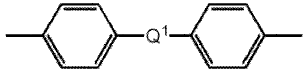
dans laquelle Q¹ est -C_{y}H_{2y}- dans lequel y est un nombre entier de 1 à 5, et
dans lequel le polyétherimide a moins de 10 parties par milliard, de préférence moins de 9 parties par milliard de sel de métal dialcalin résiduel d'un composé aromatique à fonction dihydroxy, du composé aromatique à fonction dihydroxy correspondant, du sel monosubstitué correspondant du composé aromatique à fonction dihydroxy ou d'une combinaison comprenant au moins l'un des composés susmentionnés.
